# EUROPEAN PATENT APPLICATION

(11) **EP 3 524 210 A2**
(43) Date of publication of application: **14.08.2019**
(21) Application number: 18215433.6
(22) Date of filing: 21.12.2018
(51) Int. Cl.: A61F 2/88, A61F 2/07

(54) **RADIOPAQUE MARKERS ON A MEDICAL DEVICE**

(30) Priority: 22.12.2017 US 201762609917 P
(71) Applicant: Cook Medical Technologies, LLC, Bloomington, IN 47404 (US)
(72) Inventor: Woong, Kim, West Lafayette, IN Indiana 47906 (US); Kratzberg, Jarin A., Lafayette, IN Indiana 47909 (US); Svendsen, Mark, Bloomington, IN Indiana 47401 (US); Roeder, Blayne A., Bloomington, IN Indiana 47401 (US); Rasmussen, Erik E., 4200 Slagelse (DK); Soderlind, Krista Hald, 2880 Bagsvaerd (DK); Bro, Kristoffer Vandborg, 2450 Copenhagen (DK); Christensen, Mette Louise, 2500 Valby (DK)
(74) Representative: Leach, Sean Adam

(57) **Abstract**

Systems and techniques for positioning radiopaque markings on medical stent devices are provided. Specific combinations of adjacent radiopaque markings and overlapping radiopaque markings on the stent devices are presented to provide specific feedback information to an administrator working to guide the stent devices through vessels.

## Description

### PRIORITY CLAIM

This invention claims the benefit of priority of U.S. Provisional Application Serial No. 62/609,917, entitled "Radiopaque Markers on a Medical Device," filed December 22, 2017, the disclosure of which is hereby incorporated by reference in its entirety.

### BACKGROUND

The present disclosure relates generally to medical devices, systems, and methods, and particularly, to systems and techniques for positioning radiopaque markings on medical stent devices. Stent devices may be comprised of a metal or alloy material shaped into a tube form, that are inserted into vessels or passageways for various repair purposes. For example, once inserted into a vessel, the stent device may be used to expand the vessel in an angioplasty procedure to restore increased blood flow through a previously narrowed vessel. Stent devices may also be used to strengthen and stabilize weakened vessel walls to prevent the vessel from rupturing. Stent devices may also be used as part of a procedure to treat aortic dissections, where the stent device is expanded within either a true lumen or a false lumen of the aorta to re-attach an aortic wall dissection.

Imaging techniques that utilize the attachment of radiopaque markings on the stent device are used to identify the positioning and location of the stent device as the stent device traverses through a vessel. Understanding the positioning and location of the stent device is helpful to an administrator controlling the insertion of the stent device to guide the stent device to a desired location and in a desired orientation. However, with the existing simple radiopaque marking techniques, there may be a limit to the amount of helpful information the radiopaque markings can convey back to the administrator.

The present disclosure looks to address the shortcomings of the existing simple radiopaque marking techniques.

### SUMMARY

Radiopaque marking systems and methods of implementing the different radiopaque marking combinations made available by the radiopaque marking system are disclosed. In one example, a stent device is disclosed comprised of a strut wire, a coil threaded onto the strut wire, and a ring marker threaded onto the strut wire and positioned adjacent to the coil, wherein the coil and the ring marker are positioned on the stent device to represent feedback information related to the stent device.

In another example, a method of producing a stent device is disclosed comprising threading a coil around a strut, threading a ring marker around the strut to be adjacent to the coil, attaching a rod strut to the stent device, attaching a ring strut to the stent device, and wherein the coil, the ring marker, the rod strut, and the ring strut are positioned on the stent device to represent feedback information related to the stent device. According to some embodiments, the rod strut and the ring struts are formed to be a single continuous structure, whereas in other embodiments the rod strut and the ring struts are separate and distinct structures.

Systems and techniques for positioning radiopaque markings on medical stent devices are provided. Specific combinations of adjacent radiopaque markings and overlapping radiopaque markings on the stent devices are presented to provide specific feedback information to an administrator working to guide the stent devices through vessels. Other systems, methods, features and advantages of the disclosed features will be, or will become, apparent to one with skill in the art upon examination of the following figures and detailed description. It is intended that all such additional systems, methods, features and advantages be within the scope of this disclosure, and be encompassed by the following claims.

### BRIEF DESCRIPTION OF THE DRAWINGS

The disclosed features may be better understood with reference to the following drawings and description. The components in the figures are not necessarily to scale, emphasis instead being placed upon illustrating the principles of the disclosed features. Moreover, in the figures, like referenced numerals designate corresponding parts throughout the different views.
**FIG. 1** shows a side view of an exemplary first embodiment for a radiopaque marking system.
**FIG. 2** shows a side view of an exemplary second embodiment for the radiopaque marking system.
**FIG. 3** shows a cross sectional side view and a corresponding front view from a distal end of exemplary radiopaque markers.
**FIG. 4** shows a top view of exemplary radiopaque marking configurations according to the first embodiment of the radiopaque marking system.
**FIG. 5** shows a top view of exemplary radiopaque marking configurations according to the first embodiment of the radiopaque marking system.
**FIG. 6** shows a top view of an exemplary radiopaque marking configuration according to the second embodiment of the radiopaque marking system.
**FIG. 7** shows a top view and a rotated view of an exemplary radiopaque marking configuration.
**FIG. 8** shows a graph depicting brightness gains under fluoroscopy imaging for a ring marker only configuration, coil only configuration, and a threaded combination of a ring marker and coil.
**FIGS. 9A-9B** show perspective views of exemplary configurations for radiopaque marking configurations of a side branch of a stent-graft.

### DETAILED DESCRIPTION

Radiography, fluoroscopy, and X-ray computed tomography (X-ray CT) are examples of X-ray based imaging techniques for viewing the internal workings of an object, often times a human body. The X-ray is a form of electromagnetic radiation generated by an X-ray generator and projected towards the object. As the X-rays pass through the object, some of the X-rays are absorbed to varying degrees by the different materials within the object based on various factors such as density and composition. The X-rays that make it through the object without being absorbed are detected by an X-ray detector and used to create X-ray images. Fluoroscopy is an X-ray based imaging technique that produces live, moving, images of the object that are comprised of a plurality of static X-ray images. X-ray CT is an X-ray based imaging technique that utilizes image processing techniques on X-ray images to produce a 3-dimensional image of the object.

When applying X-ray imaging techniques to monitor the insertion of a medical device into a human body, understanding the location and orientation of the medical device is desired. To provide the feedback information on the location and orientation of the medical device as it is being inserted, radiopaque markers may be included at strategic locations on the medical device. For exemplary purposes, this disclosure will reference the monitoring of a stent type of medical device into the human body during a fluoroscopy imaging procedure, although the radiopaque marking monitoring features disclosed herein may be applicable to other types of medical devices that are inserted into the human body and other types of X-ray based imaging. The stent device may be, for example, a stent-graft or endograft.

Radiopaque markers are made from a material (e.g., platinum or gold) that prevents X-rays from passing through. This results in the radiopaque markers standing out in a bright contrast in the resulting X-ray image. Radiopaque markers may be placed at specific locations on an endograft to identify specific features of the endograft. For example, radiopaque markers may be positioned along a side branch on the endograft to more easily identify these features on the resulting X-ray image.

As the number of radiopaque markers that are included on the endograft increases, the ability of an administrator to differentiate the intended feedback information from the radiopaque markers may become more difficult. As a solution to this problem, a radiopaque marking strategy is provided that combines the use of coil, ring, and/or other radiopaque markers at strategic locations on an endograft to represent specific information to be read by an administrator of the endograft during an X-ray imaging process. The radiopaque markers are threaded onto the endograft strut directly to provide both intuitive pseudo-3D information of the side branch portions on the endograft, while also reducing a sewing time for threading the radiopaque markers onto the endograft strut, and also reducing a diameter profile of the resulting endograft. The radiopaque marking strategy includes embodiments where different types of radiopaque markers (e.g., coils and ring markers) are positioned adjacent to each other in specific combinations representing specific predetermined feedback information, as well as embodiments where different types of radiopaque markers are positioned overlapping each other in specific combinations representing specific predetermined feedback information. The placement of the radiopaque markers in the specific combinations are highlighted visual accent points viewable in the resulting X-ray images. The feedback information are representative of labeling specific endograft portions, as well as indicating orientation of the specific endograft portions.

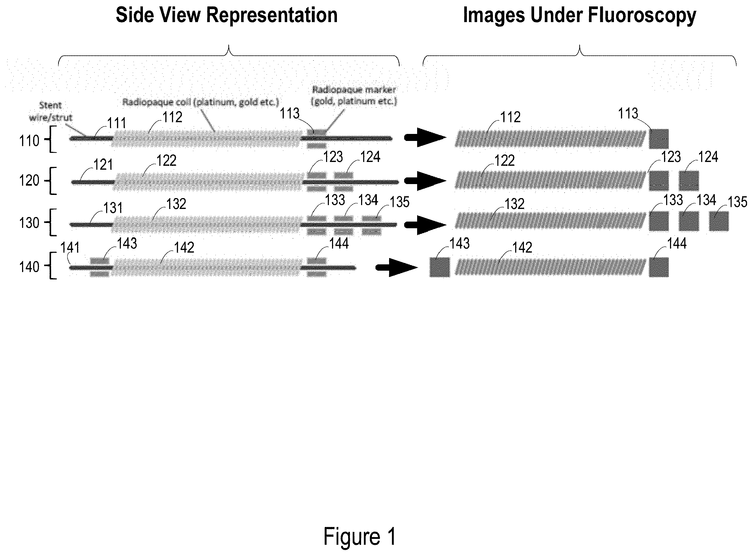

**FIG. 1** shows a side view of four exemplary combinations of radiopaque coils and radiopaque ring markers under an exemplary first embodiment. The side view shown in **FIG. 1** is a close up view on a single strut wire on an endograft, where ring markers are shown as cross sectional views to expose the strut wire around which the ring markers are secured, including but not limited using knots, glue, frictional fits, clamps, or other securing members.

A first combination 110, a second combination 120, a third combination 130, and a fourth combination 140 are all shown in **FIG. 1** to include specific combinations of radiopaque coils and radiopaque ring markers, that are secured onto the individual strut wires. The radiopaque coils and radiopaque ring markers are made from a composite material that inhibits X-rays from passing through, such as platinum or gold. **FIG. 1** also shows a resulting exemplary image under fluoroscopy for each of the first combination 110, the second combination 120, the third combination 130, and the fourth combination 140.

The first combination 110 includes a coil 112 and a ring marker 113 installed adjacent to each other along a strut wire 111 of the endograft. A resulting image of the first combination 110 under fluoroscopy shows how the coil 112 and the ring marker 113 are visibly highlighted to clearly show the coil 112 adjacent to the ring marker 113. Although the strut wire 111 is illustrated to be non-visible in the resulting image under fluoroscopy, according to some embodiments the strut wire 111 may be visible to varying degrees in the resulting image under fluoroscopy based on the fluoroscopy imaging technique being used. The space between the coil 112 and ring marker 113 may be an empty space, or occupied by a non-radiopaque marker, to better distinguish between the individual coil 112 and ring marker 113 that combines to form the distinct first combination 110 under the resulting fluoroscopy image. Alternatively, according to some embodiments the space between the coil 112 and the ring marker 113 may be removed so that the coil 112 and the ring marker 113 are adjacent to each other. By removing the space, the first combination 110 is modified into another unique form under the resulting fluoroscopy image.

The second combination 120 includes a coil 122, a ring marker 123, and a ring marker 124 that are installed adjacent to each other along a strut wire 121 of the endograft. A resulting image of the second combination 120 under fluoroscopy shows how the coil 122 and the two ring markers 123, 124 are visibly highlighted to clearly show the coil 122 adjacent to the ring marker 123, and the ring marker 123 adjacent to the ring marker 124.

Although the strut wire 121 is illustrated to be non-visible in the resulting image under fluoroscopy, according to some embodiments the strut wire 121 may be visible to varying degrees in the resulting image under fluoroscopy based on the fluoroscopy imaging technique being used. The space between the coil 122 and the ring marker 123, and/or the space between the ring marker 123 and the ring marker 124, may be an empty space, or occupied by a non-radiopaque marker, to better distinguish between the individual radiopaque members that comprise the second combination 120 (e.g., coil 122, ring marker 123, ring marker 124) under the resulting fluoroscopy image. Alternatively, according to some embodiments a space between the individual radiopaque members that comprise the second combination 120 (e.g., coil 122, ring marker 123, ring marker 124) may be removed so that one or more of the individual radiopaque members that comprise the second combination 120 (e.g., coil 122, ring marker 123, ring marker 124) are adjacent to each other. By removing a space, the second combination 120 is modified into another unique form under the resulting fluoroscopy image.

The third combination 130 includes a coil 132, a ring marker 133, a ring marker 134, and a ring marker 135, that are installed adjacent to each other along a strut wire 131 of the endograft. A resulting image of the third combination 130 under fluoroscopy shows how the coil 132 and the three ring markers 133, 134, 135 are visibly highlighted to clearly show the coil 132 adjacent to the ring marker 133, the ring marker 133 adjacent to the ring marker 134, and the ring marker 134 adjacent to the ring marker 135.

Although the strut wire 131 is illustrated to be non-visible in the resulting image under fluoroscopy, according to some embodiments the strut wire 131 may be visible to varying degrees in the resulting image under fluoroscopy based on the fluoroscopy imaging technique being used. The space between the coil 132 and the ring marker 133, and/or the space between the ring marker 133 and the ring marker 134, and/or the space between the ring marker 134 and the ring marker 135, may be an empty space, or occupied by a non-radiopaque marker, to better distinguish between the individual radiopaque members that comprise the third combination 130 (e.g., coil 132, ring marker 133, ring marker 134, ring marker 135) under the resulting fluoroscopy image. Alternatively, according to some embodiments a space between the individual radiopaque members that comprise the third combination 130 (e.g., coil 132, ring marker 133, ring marker 134, ring marker 135) may be removed so that one or more of the individual radiopaque members that comprise the third combination 130 (e.g., coil 132, ring marker 133, ring marker 134, ring marker 135) are adjacent to each other. By removing a space, the third combination 130 is modified into another unique form under the resulting fluoroscopy image.

The fourth combination 140 includes a coil 142, a ring marker 143, and a ring marker 144 that are installed adjacent to each other along a strut wire 141 of the endograft. A resulting image of the fourth combination 140 under fluoroscopy shows how the coil 142 and the two ring markers 143, 144 are visibly highlighted to clearly show the ring marker 143 installed on one end of the coil 142, and the ring marker 144 installed on the opposite end of the coil 142.

Although the strut wire 141 is illustrated to be non-visible in the resulting image under fluoroscopy, according to some embodiments the strut wire 141 may be visible to varying degrees in the resulting image under fluoroscopy based on the fluoroscopy imaging technique being used. The space between the ring marker 143 and the coil 142, and/or the space between the coil 142 and the ring marker 144, may be an empty space, or occupied by a non-radiopaque marker, to better distinguish between the individual radiopaque members that comprise the fourth combination 140 (e.g., coil 142, ring marker 143, ring marker 144) under the resulting fluoroscopy image. Alternatively, according to some embodiments a space between the individual radiopaque members that comprise the fourth combination 140 (e.g., coil 142, ring marker 143, ring marker 144) may be removed so that one or more of the individual radiopaque members that comprise the fourth combination 140 (e.g., coil 142, ring marker 143, ring marker 144) are adjacent to each other. By removing a space, the fourth combination 140 is modified into another unique form under the resulting fluoroscopy image.

The combinations illustrated in **FIG. 1** are not limiting, and any number of combinations of ring markers, coils, spaces, and/or non-radiopaque members are within the scope of this disclosure.

Each combination of adjacent radiopaque coils and radiopaque ring markers shown in **FIG. 1** may represent feedback information such as a specific endograft feature (e.g., a side arm) or an outline of the endograft itself for orientation purposes. Other combinations of adjacent radiopaque coils and radiopaque ring markers are also within the scope of the first embodiment for representing feedback information not specifically discussed. The radiopaque coils may be made from a same or different material as the radiopaque ring markers. The radiopaque coils provide a different density/composition due to its coiled shape as the radiopaque markers, and therefore result in a different overall brightness compared to the solid radiopaque ring markers on the resulting fluoroscope image.

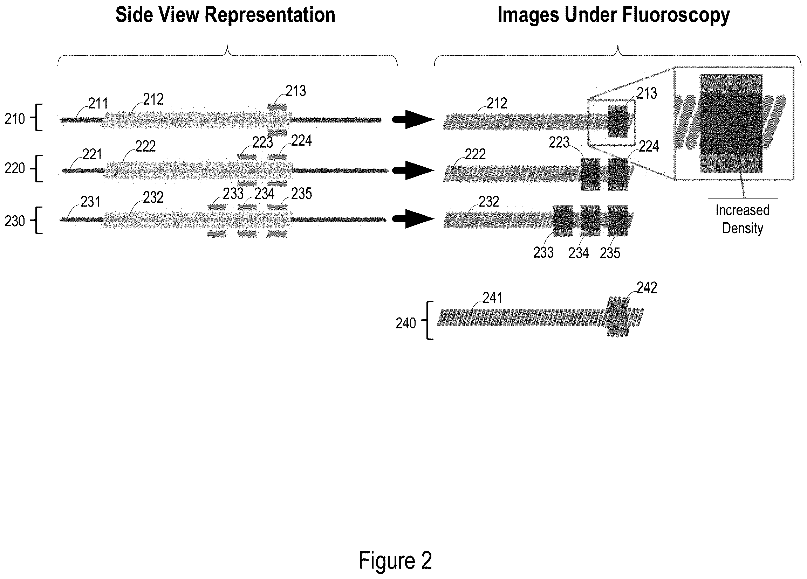

**FIG. 2** shows a side view of three exemplary combinations of radiopaque coils and radiopaque ring markers under an exemplary second embodiment. The side view shown in **FIG. 2** is a close up view on a single strut wire on an endograft, where ring markers are shown as cross sectional views to expose the coils around which the ring markers are installed over.

A first combination 210, a second combination 220, and a third combination 230 are all shown in **FIG. 2** to include specific combinations of radiopaque coils and radiopaque ring markers, that are secured onto the individual strut wires. The radiopaque coils and radiopaque ring markers are made from a composite material that inhibits X-rays from passing through, such as platinum or gold. **FIG. 2** also shows a resulting image under fluoroscopy for each of the first combination 210, the second combination 220, and the third combination 230.

The first combination 210 includes a coil 212 and a ring marker 213, where the coil is installed along a strut wire 211 of the endograft, and the ring marker 213 is installed over the coil 212. A resulting image of the first combination 210 under fluoroscopy shows how the coil 212 and the ring marker 213 are visibly highlighted to clearly show the ring marker 213 installed to overlap with the coil 212. Under a magnified view of the fluoroscopy image of the first combination 210, the density of the ring marker 213 overlapping the coil 212 is shown as being darker on the fluoroscopy image than either the coil 212 or the ring marker 213 alone.

Although the strut wire 211 is illustrated to be non-visible in the resulting image under fluoroscopy, according to some embodiments the strut wire 211 may be visible to varying degrees in the resulting image under fluoroscopy based on the fluoroscopy imaging technique being used.

The second combination 220 includes a coil 222, a ring marker 223, and a ring marker 224. The coil 222 is installed on a strut wire 221 of the endograft, and the ring markers 223, 224 are installed to overlap with the coil 222. A resulting image of the second combination 220 under fluoroscopy shows how the coil 222 and the two ring markers 223, 224 are visibly highlighted to clearly show the two ring markers 223, 224 installed to overlap the coil 222, where the overlapping portions in the fluoroscopy image are shown to be darker than either the coil 222 or the ring markers 223, 224 alone.

Although the strut wire 221 is illustrated to be non-visible in the resulting image under fluoroscopy, according to some embodiments the strut wire 221 may be visible to varying degrees in the resulting image under fluoroscopy based on the fluoroscopy imaging technique being used. The space between the ring marker 223 and the ring marker 224, may be an empty space, or occupied by a non-radiopaque marker, to better distinguish between the ring marker 223 and the ring marker 224 that overlap the coil 222, under the resulting fluoroscopy image. Alternatively, according to some embodiments the space between the ring marker 223 and the ring marker 224 may be removed so that they are adjacent to each other. By removing the space, the second combination 220 is modified into another unique form under the resulting fluoroscopy image.

The third combination 230 includes a coil 232, a ring marker 233, a ring marker 234, and a ring marker 235. The coil 232 is installed on a strut wire 231 of the endograft, and the ring markers 233, 234, 235 are installed to overlap with the coil 231. A resulting image of the third combination 230 under fluoroscopy shows how the coil 232 and the three ring markers 233, 234, 235 are visibly highlighted to clearly show the three ring markers 233, 234, 235 installed to overlap the coil 232, where the overlapping portions in the fluoroscopy image are shown to be darker than either the coil 232 or the ring markers 233, 234, 235 alone.

Although the strut wire 231 is illustrated to be non-visible in the resulting image under fluoroscopy, according to some embodiments the strut wire 231 may be visible to varying degrees in the resulting image under fluoroscopy based on the fluoroscopy imaging technique being used. The space between the ring marker 233 and the ring marker 234, and/or the space between the ring marker 234 and the ring marker 235, may be an empty space, or occupied by a non-radiopaque marker, to better distinguish between the ring marker 233, the ring marker 234, and the ring marker 235 that overlap the coil 232, under the resulting fluoroscopy image. Alternatively, according to some embodiments the space between the ring marker 233, the ring marker 234, and the ring marker 235 may be removed so that they are adjacent to each other. By removing the space, the third combination 230 is modified into another unique form under the resulting fluoroscopy image.

The combinations illustrated in **FIG. 2** are not limiting, and any number of combinations of ring markers, coils, spaces, and/or non-radiopaque members are within the scope of this disclosure.

Each combination of overlapping radiopaque ring markers and radiopaque rings shown in **FIG. 2** may represent feedback information such as a specific endograft feature (e.g., a side arm) or an outline of the endograft itself for orientation purposes. Other combinations of overlapping radiopaque coils and radiopaque ring markers are also within the scope of the second embodiment for representing feedback information not specifically discussed. For example, a fourth combination (shown under fluoroscopy only) includes a coil 241 and ring marker 242, where the ring marker 242 is installed over a strut wire (not illustrated) while being within the coil 241.

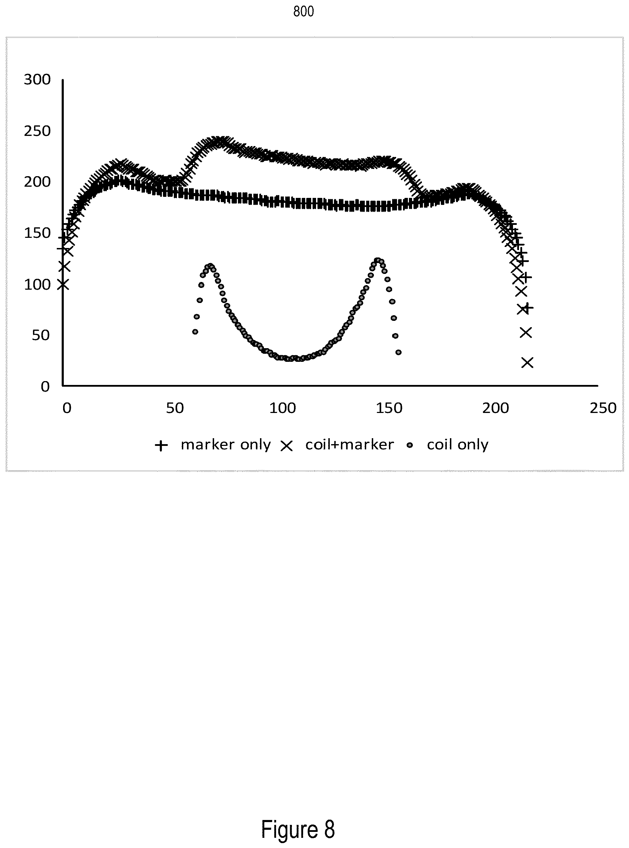

The radiopaque coils may be made from a same or different material as the radiopaque ring markers. The radiopaque coils may provide a different level of fluoroscopic brightness from that of the radiopaque markers due to its coiled shape configuration versus the solid band of the radiopaque marker even if both radiopaque coil and radiopaque markers are made from the same material with a similar path length of x-ray transmission. For example, when brightness profile sampling is performed across the ring marker only, the coil only, and the combination of overlapping ring marker and coil, the overall gain from the combination of the threaded ring marker and coil is about a 20% increase in both a maximum and an average in the "brightness" over the ring marker only or coil only. This gain is illustrated by graph 800 shown in **FIG. 8****.**

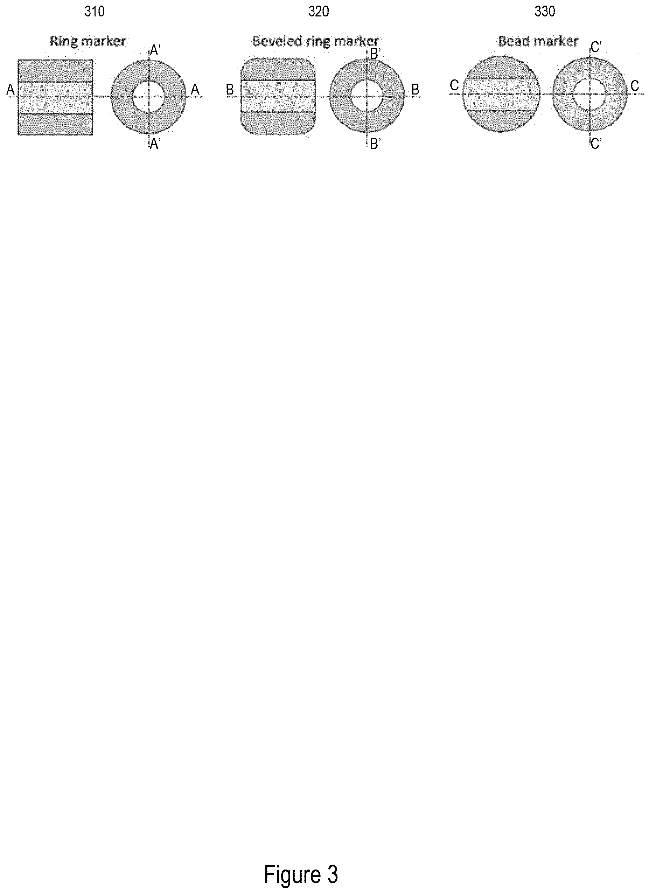

**FIG. 3** shows a cross sectional view of three exemplary designs for a radiopaque ring marker. A first ring marker 310 is shown to have perpendicular, or substantially perpendicular, edges. Looking into the first ring marker 310 from a distal end taken along the A-A line, shows a cross sectional view of the first ring marker 310. The cross sectional view of the first ring marker 310 shows a hollow section in the middle of the first ring marker 310 through which the first ring marker 310 is secured onto a strut wire of an endograft.

A second ring marker 320 is shown to have beveled, or substantially beveled, edges. Looking into the second ring marker 320 from a distal end taken along the B-B line, shows a cross sectional view of the second ring marker 320. The cross sectional view of the second ring marker 320 shows a hollow section in the middle of the second ring marker 320 through which the second ring marker 320 is secured onto a strut wire of an endograft.

A third ring marker 330 is shown to have a rounded bead-like shape with no edges. Looking into the third ring marker 330 from a distal end taken along the C-C line, shows a cross sectional view of the third ring marker 330. The cross sectional view of the third ring marker 330 shows a hollow section in the middle of the third ring marker 330 through which the third ring marker 330 is secured onto a strut wire of an endograft.

Other embodiments may use ring markers having different shapes. Ring marker shapes having rounded edges may be more easily secured onto the strut wires, and/or may prevent damaging inner linings of vessels through which the endografts travel along or the medical device itself.

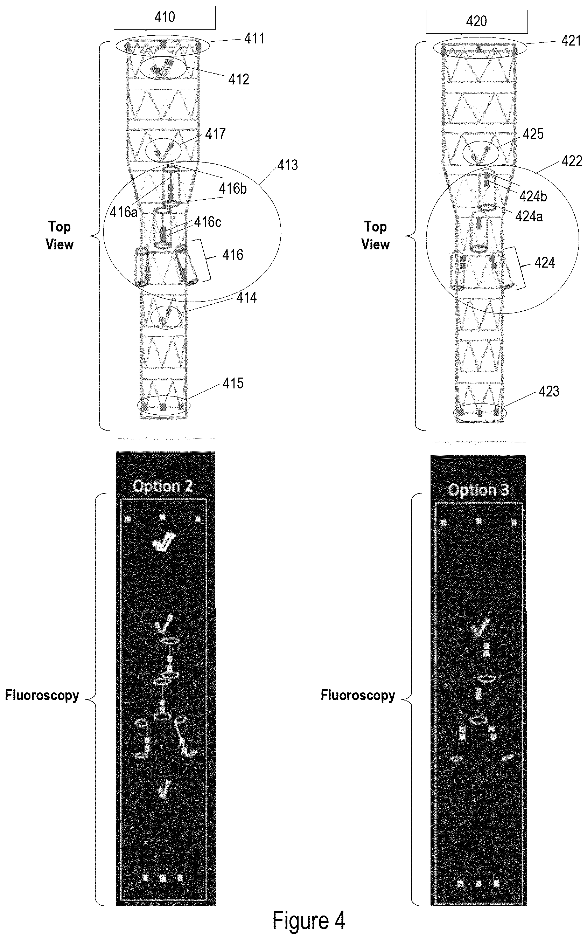

**FIG. 4** shows a top view and resulting fluoroscopy image for two different patterns of adjacent radiopaque markers according to the first embodiment. Each pattern may further be comprised of one or more sub-combination of adjacent radiopaque markers.

A first pattern 410 includes a first sub-combination 411 comprised of three ring markers secured onto different locations of a z-strut wire of an endograft. The first pattern 410 further includes a second sub-combination 412 comprised of two sets of markers in a "check mark" configuration secured onto a z-strut, where each set is comprised of ring markers of different sizes (e.g., different diameters) secured one behind the other (i.e., one is secured to the front (anterior) of the endograft and the other is secured to the back (posterior) of the endograft). The use of two sets of markers in the check mark configuration gives an operator even more precise control over orientation of the medical device as it is being inserted and moved within the subject. This is because having the two sets of markers in the check mark configuration is able to show a displacement between the two check mark markers during slight rotations of the medical device, thus giving the operator the ability to have finer ability to discern exactly what the correct orientation should be as compared to just a single check mark. In contrast, under some circumstances when the medical device is slightly rotated one way or the other with only the single check mark, the resulting image may be displayed as the same single check mark without as much discernment as to how the rotation translated to the endograft.

The first pattern 410 further includes a single check mark configuration 417 positioned above a third sub-combination 413. The third sub-combination 413 includes four branch configurations 416, where each branch configuration 416 includes a strut rod 416a, a strut ring 416b at each end of the strut rod 416a, and two ring markers 416c attached around the strut rod 416a. Each branch configuration 416 represents feedback information identifying a branch structure, where the branch configuration 416 promotes the ability to see each branch structure in the endograft individually by providing an outline of an entire branch (minus, for example, a side due to two-dimensional imaging). The two strut rings 416b together with the strut rod 416a may be a single continuous piece, and work together to identify an end (e.g., distal or proximal) of the branch structure. The strut rod 416a further provides added visibility of one side and distal end of a branch by utilizing the ring markers 416c to be threaded along the strut rod 416a to be closer to one of the ends of the strut rod 416a. The combination and relative positioning of the components that comprise the branch configuration 416 further provides an operator to view a relative length, width, and orientation of the branches in space.

The first pattern 410 further includes another single check mark configuration 414 secured onto a z-strut at a position below the third sub-combination 413, where the single check mark configuration 414 is comprised of ring markers of different sizes (e.g., different diameters) secured adjacent to each other. The first pattern 410 further includes a fourth sub-combination 415 comprised of three ring markers secured onto different locations on a z-strut wire of the endograft. In 410, the use of the second sub-combination 412 positioned on the top of the endograft, the set of branch markers in the third sub-combination 413 in the middle of the endograft, and the single check mark configuration 414 positioned at the bottom of the endograft allows the user to see the relative rotational orientation of the endograft throughout the entire length of the device. The resulting fluoroscopy image of the first pattern 410 is shown below the top view, and shows the contrasting display of the radiopaque coil and radiopaque ring markers.

A second pattern 420 includes a first sub-combination 421 comprised of three ring markers secured onto different locations of a z-strut wire of an endograft. The second pattern 420 further includes a single check mark configuration 425 positioned above a second sub-combination 422. The second sub-combination 422 includes four branch configurations 424, where each branch configuration 424 includes a single strut ring 424a at one distal end, and two ring markers 424b attached at an opposite end from the ring marker 424a. Each branch configuration 424 represents feedback information identifying a branch structure, where the branch configuration 424 promotes the ability to see each branch structure in the endograft individually by providing an outline of an entire branch (minus, for example, a side due to two-dimensional imaging). Compared to the first pattern 410, the second pattern 420 includes fewer components. Even so, although the branch configuration 424 does not include distinct strut rods, the combination and relative positioning of the strut rings 424a and ring markers 424b still offer feedback information to identify, for example, a proximal and/or distal end of a branch as well as a relative length, width, and orientation of a branch in space.

The second pattern 420 further includes a third sub-combination 423 comprised of three ring markers secured onto different locations on a z-strut wire of the endograft. In the second pattern 420, the use of the single check mark configuration 425 at the top of the endograft and second sub-combination 422 in the middle of the endograft, allows the user to see the relative rotational orientation of the endograft throughout the part of the length of the device. The resulting fluoroscopy image of the second pattern 420 is shown below the top view, and shows the contrasting display of the radiopaque coil and radiopaque ring markers.

The location and design of the radiopaque coils, radiopaque ring markers, spaces between radiopaque markers (e.g., empty space or non-radiopaque materials such as glue or other solid materials), as well as the strut rods and strut rings, serve to provide feedback information by identifying specific features and/or orientation of the endograft.

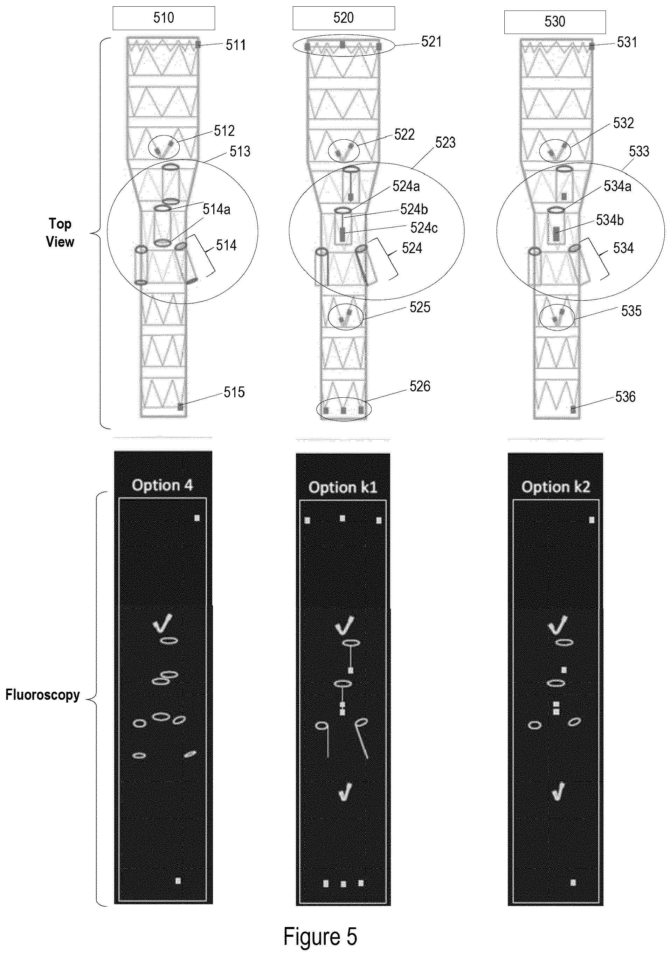

**FIG.** 5 shows a top view and resulting fluoroscopy image for three different patterns of adjacent radiopaque markers according to the first embodiment. Each pattern may further be comprised of one or more sub-combination of adjacent radiopaque markers.

A first pattern 510 includes a single ring marker 511 secured onto a z-strut wire of an endograft at a distal end. The first pattern 510 further includes a single check mark configuration 512 comprised of ring markers of different sizes installed adjacent to each other, where the check mark configuration 512 is positioned above a first sub-combination 513. The first sub-combination 513 comprises four branch configurations 514, where each branch configuration 514 includes a strut ring 514a at each distal end. Each branch configuration 514 represents feedback information identifying a branch structure, where the branch configuration 514 promotes the ability to see each branch structure in the endograft individually by providing an outline of an entire branch (minus, for example, a side due to two-dimensional imaging). With the ring struts 514a positioned at the distal ends of the branch configurations 514, feedback information to identify, for example, a proximal and/or distal end of a branch as well as a relative length, width, and orientation of a branch in space is provided.

The first pattern 510 further includes another single ring marker 515 secured onto a z-strut wire of the endograft at a distal end. The resulting fluoroscopy image of the first pattern 510 is shown below the top view, and shows the contrasting display of the radiopaque coil and radiopaque ring markers.

A second pattern 520 includes a first sub-combination 521 comprised of three ring markers secured onto different locations of a z-strut wire of an endograft at a distal end. The second pattern 520 further includes a single check mark configuration 522 comprised of ring markers of different sizes installed adjacent to each other, where the check mark configuration 522 is positioned above a second sub-combination 523. The second sub-combination 523 comprises four branch configurations 524, where each branch configuration 524 includes some combination of a strut ring 524a, a strut rod 524b, and ring markers 524c, where the ring markers 524c are threaded onto the strut rod 524b. Each branch configuration 524 represents feedback information identifying a branch structure, where the branch configuration 524 promotes the ability to see each branch structure in the endograft individually by providing an outline of an entire branch (minus, for example, a side due to two-dimensional imaging). With the combination and relative positioning of the strut ring 524a positioned at a proximal end of the branch configurations 524, the strut rod 524b, and the combination of ring markers 524c, feedback information to identify, for example, a proximal and/or distal end of a branch as well as a relative length, width, and orientation of a branch in space is provided. Having the ring markers 524c at the edges of some but not other branch configurations 524, provides a way of having better distinguishing between the branch structures.

The second pattern 520 further includes another single check mark configuration 525 comprised of ring markers of different sizes installed adjacent to each other, where the check mark configuration 525 is positioned below the second sub-combination 523. The second pattern 520 further includes a third sub-combination 526 comprised of three ring markers secured onto different locations on a z-strut wire of the endograft. The resulting fluoroscopy image of the second pattern 520 is shown below the top view, and shows the contrasting display of the radiopaque coil and radiopaque ring markers.

A third pattern 530 includes a single ring marker 531 secured onto a z-strut wire of an endograft. The third pattern 530 further includes a single check mark configuration 532 comprised of ring markers of different sizes installed adjacent to each other, where the check mark configuration 532 is positioned above a first sub-combination 533. The first sub-combination comprises four branch configurations 534, where each branch configuration 534 includes some combination of a strut ring 534a and ring markers 534b. Each branch configuration 534 represents feedback information identifying a branch structure, where the branch configuration 534 promotes the ability to see each branch structure in the endograft individually by providing an outline of an entire branch (minus, for example, a side due to two-dimensional imaging). With the combination and relative positioning of the strut ring 534a positioned at a proximal end of the branch configurations 534, and the combination of ring markers 534b, feedback information to identify, for example, a proximal and/or distal end of a branch as well as a relative length, width, and orientation of a branch in space is provided. Having the ring markers 534b at the edges of some but not other branch configurations 534, provides a way of having better distinguishing between the branch structures. The ring markers 534b also provides a user with important features, such as identifying a proximal end of a branch structure so that a path to inserting the endograft into the branch structure is shown.

The third pattern 530 further includes another single check mark configuration 535 comprised of ring markers of different sizes installed adjacent to each other, where the check mark configuration 535 is positioned below the first sub-combination 533. The third pattern 530 further includes another single ring marker 536 secured onto a z-strut wire of an endograft. The resulting fluoroscopy image of the third pattern 530 is shown below the top view, and shows the contrasting display of the radiopaque coil and radiopaque ring markers.

The location and design of the radiopaque coils, radiopaque ring markers, spaces between radiopaque markers (e.g., empty space or non-radiopaque materials such as glue or other solid materials), as well as the rods and rings, serve to provide feedback information by identifying specific features and/or orientation of the endograft.

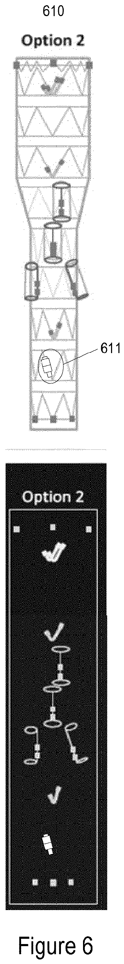

**FIG. 6** shows a top view and resulting fluoroscopy image for a first pattern 610 of adjacent radiopaque markers according to the second embodiment. Each pattern may further be comprised of one or more sub-combination of adjacent radiopaque markers, and further including overlapping radiopaque markers.

The first pattern 610 according to the second embodiment is similar to the first pattern 410 according to the first embodiment. In addition, the first pattern 610 according to the second embodiment includes a first sub-combination 611 comprised of a coil and two overlapping ring markers placed over a middle section of the coil. The first pattern 610 provides the ability to see each branch structure individually. The first pattern 610 provides an outline of a branch structure (except for one side). The first pattern 610 also provides added visibility of one side and distal end of the branch structure where the two ring markers are located on a branch configuration. The first pattern 610 also provides the ability to see the relative length, width, and orientation of the branch structures in space.

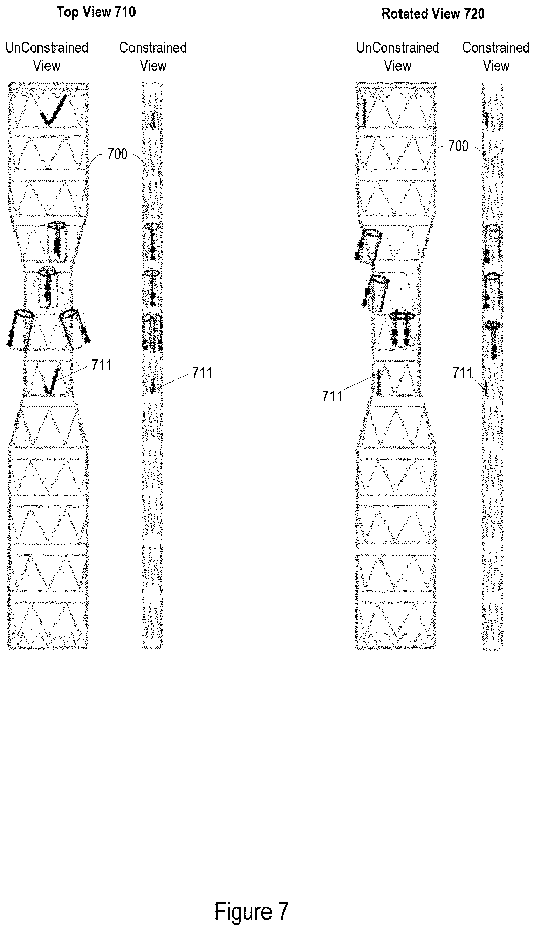

**FIG. 7** shows an exemplary endograft 700 having an exemplary radiopaque marking configuration. The endograft 700 is shown under both a top view 710 and a rotated view 720, where the rotated view 720 is a view taken of the endograft 700 that has been rotated (e.g., rotated 90 degrees) from the top view 710. **FIG. 7** further shows the endograft in an unconstrained view and a constrained view under the top view 710, and an unconstrained view and a constrained view under the rotated view 720. The unconstrained views of the endograft 700 illustrate the endograft 700 in an expanded state (e.g., for when expanded within a vessel), and the constrained views of the endograft 700 illustrate the endograft in a constricted state (e.g., for when traveling through a vessel). The comparison provided by the constrained and unconstrained views exemplify how the radiopaque marking system on the endograft 700 is configured to achieve the advantages of the feedback information by patterning the radiopaque markers in the adjacent and overlapping patterns, while still maintaining a low profile that will not adversely affect the ability of the endograft traveling within vessels. The check mark radiopaque marker 711 shown in exemplary pattern 710 is comprised of a single coil made of a radiopaque material.

Patterns that include overlapping radiopaque markers may be applied in different combinations with adjacent radiopaque markers. The combinations may include combinations of different radiopaque marker types (e.g., coils, ring markers, strut rods, strut rings), combination of different radiopaque markers that are spaced apart by empty space or non-radiopaque materials (e.g., glue, suture, or other materials with low radiopacity), combination of different radiopaque marker materials (e.g., platinum, gold, non-radiopaque materials, materials with low radiopacity, nitinol), and/or different radiopaque marker dimensions (e.g., larger radiopaque markers positioned next to smaller radiopaque markers to distinguish a unique pattern of different radiopaque markers) to achieve specific patterns of shapes and brightness on a resulting fluoroscopy image.

As noted above, **FIG. 8** shows a graph depicting brightness gains under fluoroscopy imaging for a ring marker only configuration, coil only configuration, and a threaded combination of a ring marker and coil. The numerical values represent relative brightness under experimental imaging modalities. When brightness profile sampling is performed across the marker and coil, the overall gain from the threading coil into a marker is about 20% increase in the "brightness," including both maximum and average.

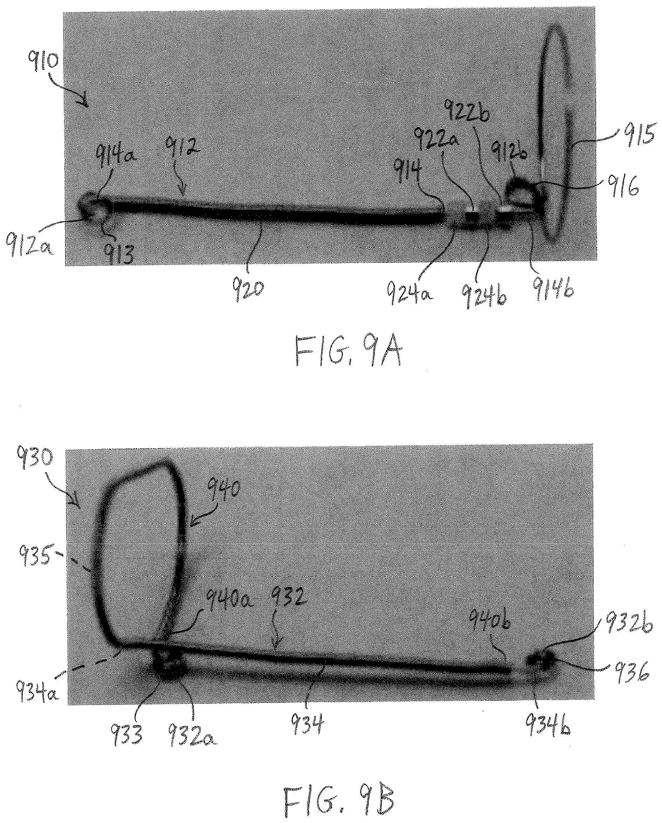

**FIGS. 9A-9B** show perspective views of exemplary configurations for radiopaque marking configurations of a side branch of an endograft. The components in **FIGS. 9A-9B** collectively form a two-part frame, including a first segment 910 and a second segment 930, which together provide support for a single side branch. This two-part frame shown in **FIGS. 9A-9B** may be an alternative structural support system to any of the side branch support structures that are depicted in the endografts shown in **FIGS. 4-7**, i.e., any individual side branch represented in **FIGS. 4-7** may instead comprise the two-part frame of **FIGS. 9A-9B****.**

In **FIG. 9A**, the first segment 910 comprises a wire 912 having a first end 912a and a second end 912b. The wire 912 spans from the first end 912a to form a first loop 913, and then continues as a relatively straight strut segment 914. The strut segment 914 extends from a first region 914a, which may be the exit location of the first loop 913, to a second region 914b. From the second region 914b, the wire 912 transitions into a ring 915, and further continues to form a second loop 916. The second loop 916 transitions to the second end 912b of the wire 912.

The ring 915 of the first segment 910 is dimensioned to encircle a fenestration in the main endograft body. Accordingly, the ring 915 and adjacent second loop 916 are disposed proximal (upstream) relative to the first loop 913. The first and second loops 913 and 916 may allow suture coupling locations for the first segment 910 to graft material of the side branch.

In the embodiment of FIG. **FIG. 9A**, the first segment 910 comprises a coil 920 that is wound about a portion, and preferably a majority, of the strut segment 914. In one non-limiting embodiment, the coil 920 comprises a radiopaque material such as platinum, although other materials may be used.

The first segment 910 further comprises at least one ring marker, such as ring markers 922a and 922b, which are disposed proximal to the coil 920. In one non-limiting embodiment, the ring markers 922a and 922b comprise a radiopaque material such as gold, although other materials may be used. In this embodiment, a plurality of spacers 924a and 924b are also used, such that the length of the strut segment 914 comprises the following sequence in a distal to proximal direction between first region 914a and second region 914b: coil 920, spacer 924a, ring marker 922a, spacer 924b, and ring marker 922b.

Advantageously, by having components arranged in this sequence, enhanced imaging of a side branch may be achieved, particularly along a longitudinal length of the side branch. As a further advantage, the spaced-apart ring markers 922a and 922b may provide a visual indication just distal to a fenestration in the main endograft when the ring 915 encircles the fenestration.

In **FIG. 9B**, the second segment 930 comprises a wire 932 having a first end 932a and a second end 932b. The wire 932 spans from the first end 932a to form a first loop 933, and then transitions into a ring 935, which in this non-limiting example comprises a "D-shape." From the ring 935, the wire 932 transitions to a relatively straight strut segment 934. The strut segment 934 extends from a first region 934a, which is generally at the end of the ring 935, to a second region 934b. From the second region 934b, the wire continues to form a second loop 936. The second loop 936 transitions to the second end 932b of the wire 932.

The ring 935 of the second segment 930 is dimensioned to encircle an open distal end of the side branch of the endograft. Accordingly, the ring 935 and adjacent first loop 933 are disposed distal to the second loop 936. The first and second loops 933 and 936 may allow suture coupling locations for the second segment 930 to graft material of the side branch.

In the embodiment of **FIG. 9B**, the second segment 930 comprises a coil 940 that is wound about the wire 932 in the location of both the ring 935 and the strut segment 934. The coil 940 has a distal region 940a that is disposed near the end of the loop 933 and a proximal region 940b that is disposed near the second region 934b of the strut segment 934 In one non-limiting embodiment, the coil 940 comprises a radiopaque material such as platinum, although other materials may be used.

Advantageously, by having a coil 940 encircle the wire 932 along a substantial part of its length, e.g., around all or substantially all of the ring 935 and strut segment 934, enhanced imaging of the side branch may be achieved, particularly at the distal opening of the side branch and along the longitudinal length of the side branch. In some embodiments, the coil 940 may comprise multiple coils, which may be spaced apart slightly from one another, e.g., to help negotiate turns of the wire 932, while still achieving the same benefits. As will be appreciated, in alternative embodiments, one or more ring markers may be disposed adjacent to the coil 940 (in a manner similar to **FIG. 9A** and other embodiments above). Further, in either of the embodiments of **FIGS. 9A-9B**, one or more ring markers may be disposed over the coils 920 or 940 as explained, for example, with respect to **FIG. 2** above.

Advantageously, the two-part frame comprising the first segment 910 and the second segment 930 cooperate to provide excellent structural support for a single side branch, while also integrating radiopaque markers in the form of coils and ring markers. The coil and ring markers are beneficially arranged in specific patterns around the wires 912 and 932 of the first and second segments 910 and 930 to provide optimal visualization of the location of the side branch, its distal opening, and the fenestation in the main body of the endograft.

While various embodiments have been described, the disclosed features are not to be restricted except in light of the attached claims and their equivalents. Moreover, the advantages described herein are not necessarily the only advantages and it is not necessarily expected that every embodiment will achieve all of the advantages described. Some embodiments may also include a fewer, or greater, number of components or steps than those specifically described and still remain within the scope of this disclosure.

## Claims

1. A stent device comprising:
a strut wire;
a coil threaded onto the strut wire;
a ring marker threaded onto the strut wire and positioned adjacent to the coil; and
wherein the coil and the ring marker are positioned on the stent device to represent feedback information related to the stent device.

2. The stent device of claim 1, further comprising:
a supplemental ring marker; and
a rod strut attached to the stent device, wherein the rod strut is threaded through the supplemental ring marker.

3. The stent device of claim 1 or 2, further comprising:
a spacer positioned between the coil and the ring marker, where the spacer is composed of a non-radiopaque material.

4. The stent device of any preceding claim, wherein the ring marker includes a rounded edge.

5. The stent device of any preceding claim, further comprising:
a supplemental ring marker; and
a supplemental coil, wherein the supplemental ring marker is positioned to overlap with at least a portion of the supplemental coil.

6. The stent device of any preceding claim, wherein an inner diameter of the ring marker is larger than a diameter of the coil.

7. The stent device of any preceding claim, wherein a radiopaque material of the coil is different from a radiopaque material of the ring marker.

8. The stent device of any preceding claim, wherein a material density of the coil is different from a material density of the ring marker.

9. A frame system for a side branch of an endograft, the system comprising:
a first segment comprising a wire, where a proximal region of the wire of the first segment forms a ring dimensioned to surround a fenestration of the endograft;
a second segment comprising a wire, where a distal region of the wire of the second segment forms a ring dimensioned to surround a distal opening of the branch side branch;
a coil threaded onto the wire of the first segment;
a ring marker threaded onto the wire of the first segment; and
a coil threaded onto the wire of the second segment.

10. The frame system of claim 9, further comprising a spacer disposed between the coil and the ring marker of the first segment.

11. The frame system of claim 9 or 10, wherein multiple ring markers are threaded onto the wire of the first segment at a location proximal to the coil of the first segment.

12. The frame system of claim 11, wherein a straight strut segment of the first segment receives, in a distal to proximal direction: the coil, a first spacer, a first ring marker, a second spacer, and a second ring marker.

13. The frame system of any of claims 9 to 12, wherein the wire of the first segment forms at least one loop, which is smaller than the ring of the first segment, and which is configured to receive a suture.

14. The frame system of any of claims 9 to 13, wherein the coil of the second segment extends around the ring and a straight strut segment.
